# EUROPEAN PATENT SPECIFICATION

(11) **EP 2 682 187 B1**
(45) Date of publication and mention of the grant of the patent: **13.09.2017**
(21) Application number: 12174574.9
(22) Date of filing: 02.07.2012
(51) Int. Cl.: B01L 3/00, C12M 1/32, G01N 33/50, G01N 33/569, G01N 35/00

(54) **Selection method and apparatus**
Auswahlverfahren und -vorrichtung
Méthode de sélection et appareil

(43) Date of publication of application: 08.01.2014
(73) Proprietor: Molecular Devices LLC, Sunnyvale, CA 94089 (US)
(72) Inventor: Robertson, Alasdair Macmillan, Bornemouth Dorset BH6 3JN (GB); Game, Stephen Martin, Bournemouth Dorset BH6 3LU (GB); Burke, Julian Francis, Winchester Hampshire SO23 9HX (GB); Truesdale, Mark Robert, Bournemouth Dorset BH6 5DZ (GB); Phillips, Anna Louise, Southampton Hampshire SO15 3HH (GB); Landau, Lee Von, Marchwood Southampton SO40 4YQ (GB); Simmonds, Jonathan, New Milton Hampshire BH25 5UH (GB)
(74) Representative: Hartley, Andrew Philip

(56) References cited:
- EP-A1- 1 502 649
- EP-A1- 2 151 689
- EP-A2- 1 742 064
- EP-A2- 1 998 162
- WO-A2-03/067904
- TEKKUMKARA T J ET AL: "Stable expression of a functional rat angiotensin II (AT1A) receptor in CHO-K1 cells: Rapid desensitization by angiotensin II", MOLECULAR AND CELLULAR BIOCHEMISTRY, NORWELL, MA, US, vol. 146, 10 May 1995 (1995-05-10), pages 79-89, XP009162960, ISSN: 0300-8177
- SPIE, PO BOX 10 BELLINGHAM WA 98227-0010 USA, May 2006 (2006-05), XP040214200,
- Manish Gupta: "EXPRESSION OF ANGIOTENSIN II TYPE I RECEPTOR ON ERYTHROID PROGENITORS OF PATIENTS WITH POST TRANSPLANT ERYTHROCYTOSIS", TRANSPLANTATION, vol. 70, 1 January 2000 (2000-01-01), pages 1188-1194, XP055054726,
- Nan Hao ET AL: "Signal-dependent dynamics of transcription factor translocation controls gene expression", Nature Structural & Molecular Biology, vol. 19, no. 1, 18 December 2011 (2011-12-18), pages 31-39, XP55175442, ISSN: 1545-9993, DOI: 10.1038/nsmb.2192

## Description

There are many situations in which it is desirable to select (that is to say choose), from a heterogeneous population, a cell colony or a single cell which has a desired characteristic.

One example is the selection of a cell colony or a single cell to form a cell line for a cell-based reporter assay. A cell-based reporter assay may, for example, be used to screen candidate compounds for new drugs. In an example of such an assay, a culture of a suitable cell line, expressing a particular protein, is exposed to a candidate compound and the cells are assessed for a marker indicating a desired interaction (direct or indirect) between the candidate compound and the protein. Cell lines for cell-based reporter assays can be generated by transfecting readily cultured cells so that the cells express functional copies of transgenic or recombinant polypeptides or proteins of interest. However, the transfection process results in a heterogeneous mix of cells and it is critical to select those in which the transfection process has resulted in the desired characteristic.

Currently, after transfection, cells are subjected to limiting dilution and plated in multi-well plates so that, statistically, it is unlikely that any well will contain more than one cell. Many wells have no cells. Clonal cell colonies that form during incubation are sub-cultured to produce a plurality of different clonal cultures, each in duplicate. For each clonal culture, one of the duplicates is tested, generally destructively, for the desired characteristic. This enables the clonal cultures (ie the untested duplicates) to be narrowed down to those which exhibit the desired characteristic. This process is inefficient because it inevitably entails the culturing of clones which turn out not to have the desired characteristic.

EP 2 151 689 A1 discloses a method of selecting cells which express high levels of a polypeptide of interest. The cells are exposed to an exogenous marker compound which associates with an endogenous reference polypeptide. Expression of the reference polypeptide is linked to expression of the polypeptide of interest. The marker compound is labelled so that cells to which the marker compound binds can be identified microscopically. In some embodiments the cells are pre-incubated with the marker compound to amplify the genes coding for the reference polypeptide and the polypeptide of interest.

The article entitled "Signal-dependent dynamics of transcription factor translocation controls gene expression", by Hao and O'Shea in Nature Structural & Molecular Biology, Vol 19, No. 1, (2012) pp31 - 39 discloses preparation of a cell line which expresses the transcription factor Msn2 labelled with yellow fluorescent protein. Translocation of the transcription factor from the cytoplasm to the nucleus is detected microscopically. The translocation to the nucleus is triggered by environmental stress or by inhibiting protein kinase A.

### Summary of the Invention

According to a first aspect of the invention, there is provided a method according to claim 1.

Preferably, when the method is applied to cultured cell colonies, the colonies will be clonal cell colonies, although the method is also applicable to non-clonal cell colonies.

The transcription factor is preferably transgenic or recombinant.

The method includes providing a plurality of cultured cell colonies or a plurality of cultured single cells in a common culture space. The common culture space is a single continuous culture space. It may comprise distinct interconnected zones, but not unconnected culture spaces such as separate wells of a multi-well plate. Hence, the common culture space may be, for example, the culture space within a single conventional Petri dish, the culture space within a single culture flask, or the culture space within a single well of a multi-well culture plate. The cell colonies or single cells are cultured together, within the common culture space, but generally they will be spatially separated from one another. The common culture space will generally contain a continuous volume of growth medium which supports all of the cultured cell colonies or all of the single cells. The growth medium may, for example, be a liquid growth medium or a semi-solid growth medium. Preferably, the culture medium will be a liquid growth medium and the cell colonies or single cells will be adherent. In general, the precise composition of the culture medium is not critical so long as the medium has all components necessary to support the cells.

Preferably, the cells of the cell colonies will undergo division when in culture. However, this is not necessary and the term "culture" as used herein includes the case that the cells are maintained alive without dividing.

Preferably, the method will be performed on cultured cell colonies rather than single cells. The cell colonies will generally be provided by preparing a suspension of single cells which are heterogeneous in terms of presence of functional copies of the transcription factor and plating the cells in the common culture space so that the cells are mostly spatially separated from one another. The cells are then cultured so as to form spatially separated cell cultures which are mostly clonal. The use of cell colonies for the method is preferred because the growth rate of the colonies can be taken into account when selecting suitable colonies.

It is possible that there will be a mixture of cell colonies and single cells in the same common culture space with the method being performed on the mixture.

The heterogeneity exhibited between different cell colonies or between different single cells may take the form of presence or absence of the functional copies of the transcription factor, or of different levels or magnitude of the functional copies of the transcription factor, and in the latter case this may include no appreciable presence of the functional copies of the transcription factor.

The method includes adding a ligand for the transcription factor to the cell colonies or to the single cells, while the cell colonies or the single cells are in the common culture space. In a preferred embodiment, the ligand is applied to the common culture space in a manner such that all of the cell colonies or all of the single cells are subjected to the ligand (albeit perhaps at different intensities or concentrations) within a relatively short period of time, for example within 10, 20 or 30 seconds.

In other preferred embodiments, individual cell colonies or individual single cells are subjected to the ligand at different times. For example, the ligand could be contained in a growth medium in an inactive form. The chemical could then be converted to an active form, in a localised manner, near an individual cell colony or single cell, by application of a suitable localised stimulus, such as, for example, a narrow beam of light.

Where the cell colonies or single cells are cultured in a liquid growth medium, all of the cell colonies or single cells may be exposed to the ligand within a relatively short time by adding to the culture medium a volume of liquid containing the ligand that is relatively large compared to the volume of the liquid medium. For example, the volume of liquid added may be between 20% to 50% of the volume of the liquid culture medium, or even greater if detachment of cells does not occur. In cases where the culture medium is a semi-solid medium, the cell colonies or single cells may be exposed to the ligand within a relatively short time by spraying a mist of a solution of the ligand onto the surface of the semi-solid medium. Alternatively, where the culture medium is a semi-solid medium, the ligand may be contained in an inactive form in the medium and activated by applying a suitable activating stimulus to the whole of the culture medium simultaneously.

It is not necessary that the concentration of the ligand to which the cell colonies or single cells are exposed is uniform.

The cell colonies or single cells may be subjected to additional interventions in addition to the addition of the ligand. One example of this is when an additional intervention is required to be able to detect the change in location of the transcription factor that is elicited by the addition of ligand. For example, the cultured cell colonies or the cultured single cells may first be incubated with a reporter chemical which enters into the cells of the colonies or into the single cells. Subsequently to this intervention, the ligand is added to the common culture space and elicits a change in activity of the transcription factor occurring on migration of the transcription factor from the cytoplasm to the nucleus of those ones of the cell colonies or single cells which have the functional copies of the transcription factor. The internalised reporter chemical allows the change in location of the transcription factor to be detected.

It may not be necessary to assess all of the cell colonies or the single cells for the change in location of the transcription factor. For example, the selection of the cell colonies or single cells may be based on the presence of two or more different desired characteristics. It is possible that a cell colony or single cell may have been eliminated because it does not possess a first desired characteristic, in which case the assessment of that colony or cell for a change in location of the transcription factor may be unnecessary.

The assessment for change in location of the transcription factor is performed while the cell colonies or the single cells are in the common culture space. No subdivision of the common culture space into unconnected culture spaces (eg such as in ring cloning) has occurred. Preferably, the cell colonies or single cells are still in culture medium when the assessment is performed.

The assessment for the change in location of the transcription factor is preferably performed using an imaging system. More preferably, the imaging system detects fluorescence or luminescence indicative of the change in location in individual ones of the cell colonies or individual ones of the single cells.

Instead of using fluorescence or luminescence imaging for the assessment of the cell colonies or single cells for the marker, Raman spectroscopy may be used. Raman spectroscopy is particularly well suited to detecting the presence of specific chemicals in microscopic systems and can be used to detect specific chemicals within viable cells.

The method comprises selecting at least one but not all of the cell colonies or of the single cells; such that those selected are expected to have functional copies of the transcription factor. The selection takes account of the assessment for change in location of the transcription factor to determine whether individual cell colonies or single cells are likely to have functional copies of the transcription factor. The selection may take account of other factors and so a cell colony or single cell having functional copies of the transcription factor may not necessarily be selected. For example, a cell colony or cell having the desired characteristic may not be selected if it does not have another desired characteristic. The selection is preferably performed by a processor. Indeed, the method is preferably performed in an automated manner by a robotic apparatus.

The method further includes providing an indication of the spatial position of at least one of the cell colonies or single cells that have been selected.

Preferably, the spatial position will be a position designated with reference to the common culture space, or to the culture vessel. In this way, the culture vessel may be moved and, by determining the change in position of the culture vessel, the new position of the cell colony or single cell may be determined. This may be useful as it allows picking of cell colonies or single cells after the culture vessel has been moved.

The indication of the spatial position could take a number of forms. A basic way of indicating the spatial position of a selected colony or single cell is simply to make a mark adjacent the position, or indicative of the position, on the culture vessel that provides the common culture space. For example, if the common culture space is the culture space of a Petri dish, a mark could be made on the underside of the dish adjacent the position of the cell colony or single cell. This mark could be a circle surrounding the colony or cell, or a ring tracing the boundary of a colony. The making of such a mark would allow the manual picking of the colony or single cell. For example, the mark may be used to place a ring or cylinder to seal against the base of the dish and surround the colony or cell. The cell colony or the single cell could then be detached using trypsin solution within the ring.

The term "picking" is used herein to mean taking a cell colony or a single cell from the culture space in a manner such that other cell colonies or single cells are not also taken in the same picking action. When picking a cell colony it is not necessary to take all of the cells making up the colony.

More preferably, however, the spatial position of a selected cell colony or single cell will be indicated by generating coordinates identifying the position. The coordinates may be XY coordinates, or XYZ coordinates, relating to the common culture space. This is particularly useful if picking is to be performed in an automated fashion.

Preferably, the method further includes picking at least one of the selected cell colonies or single cells.

The method may include determining respective boundaries of individual ones of the cell colonies or single cells. For example, in the case of a cell colony adhered to a horizontal surface, the boundary determined may be the perimeter of the area of the horizontal surface that is covered by the colony. Determination of a boundary of a cell colony may be useful as it may allow estimation of the size of, or the number of cells in, the colony. This may in turn be useful as it may allow a magnitude of the marker estimated for the colony to be normalised with regard to the size of, or number of cells in, the colony. It may also be useful to determine a boundary of a cell colony or single cell so as to facilitate picking of the colons or cell.

According to a second aspect of the invention, there is provided a computer program comprising instructions for directing a robotic apparatus to perform a method according to the first aspect of the invention. The computer program may be provided on a computer readable medium.

According to a third aspect of the invention, there is provided a robotic apparatus including a processor and a storage medium, the storage medium having stored thereon a computer program according to the second aspect of the invention, the processor being connected to the storage medium for running of the computer program on the processor.

Preferably, the method of the invention is performed using a robotic apparatus comprising: an imaging station whereat a culture vessel may be positioned; an imaging system adapted for producing at least one image covering a field of view including a plurality of cell colonies or of single cells contained in a common culture space of a culture vessel positioned at the imaging station; a processing device programmed to analyse the at least one image to assess individual cell colonies or single cells includes within the field of view for a change in location of a transcription factor, the change in location of the transcription factor being indicative of the presence of functional copies of the transcription factor; the processing device being programmed to select at least one but not all of the cell colonies or of the single cells, wherein said selection takes account of said assessment to determine expected possession of functional copies of the transcription factor, wherein the or each selected cell colony or single cell is expected to have functional copies of the transcription factor on the basis of said assessment, and the processing device being programmed to derive an indication of the spatial position of at least one of the cell colonies or of the single cells that are selected.

Preferably, the robotic apparatus includes a robotic dispenser for dispensing a fluid into a common culture space of a culture vessel positioned at the imaging station, the robotic dispenser being controlled by the processing device. In this case, the processing device of the robotic apparatus is preferably programmed to perform, in the following sequence, the steps of:
i) operating the imaging system to take a first image of the field of view including a plurality of cell colonies or single cells in a common culture space of a culture vessel positioned at the imaging station;
ii) operating the robotic dispenser to dispense into the common culture space of the culture vessel a fluid treatment comprising a ligand for the transcription factor:
iii) operating the imaging system to take a second image of the field of view including the plurality of cell colonies or single cells in the common culture space of the culture vessel positioned at the imaging station.

When the processing device is programmed to perform these steps, the analysis performed by the processing device for the change in location of the transcription factor comprises comparing the first and second images to identity changes between the two images corresponding to individual ones of the cell colonies or single cells.

The robotic apparatus preferably, comprises a detector for detecting fluorescent or luminescent light emitted by cell colonies or single cells in the field of view. The detector preferably comprises an sCMOS image sensor. Preferably, the detector comprises an image sensor having a sensing area of at least 100mm².

The robotic apparatus preferably comprises a robotic cell picker for picking individual cell colonies or individual single cells. The robotic cell picker is preferably controlled by the processing device. In this case, the processing device is programmed to operate the robotic cell picker to pick a cell colony or a single cell that has been selected by the processing device and for which an indication of the spatial position has been derived.

### Detailed Description

The following is a more detailed description, by way of example, reference being made to the appended schematic drawings in which:
Figure 1 is a perspective view of a robotic apparatus;
Figure 2 shows an imaging system of the robotic apparatus of Figure 1;
Figures 3A and 3B are white light images of cell colonies obtained using the imaging system of Figure 2;
Figure 4 is a perspective view of a cell picking head of the robotic apparatus of Figure 1;
Figure 5 shows white light images of AT1 expressing CHO K1 cell colonies at two different time points with and without treatment with FLIPR™ Calcium 5 ™ Reagent;
Figure 6 is a graph showing the effect on cell growth of FLIPR™ Calcium 5 ™ Rcagent on AT1 expressing CHO K1 cells;
Figures 7A and 7B show fluorescent images of cell colonies before and after a treatment;
Figure 8 is a graph showing fluorescent intensity over time; and
Figure 9 shows fluorescent images of cells before and after a treatment.

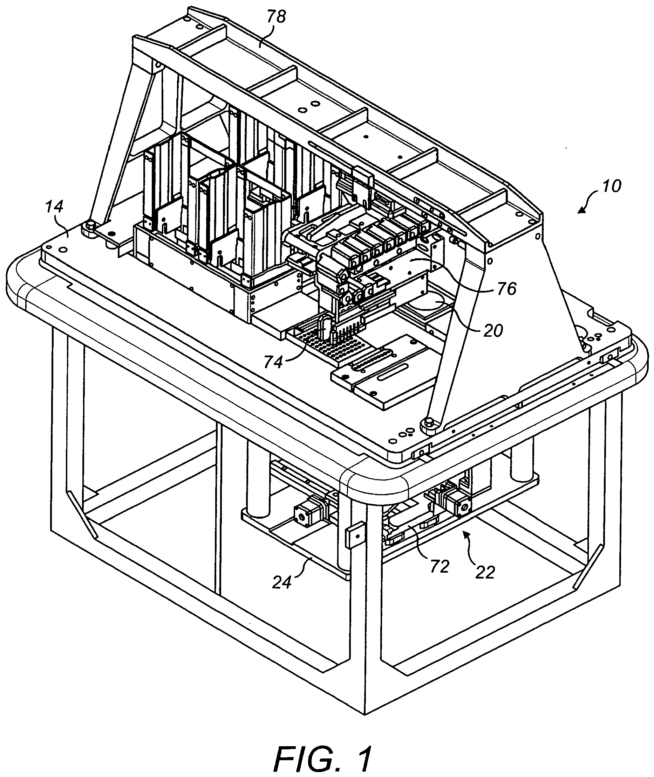

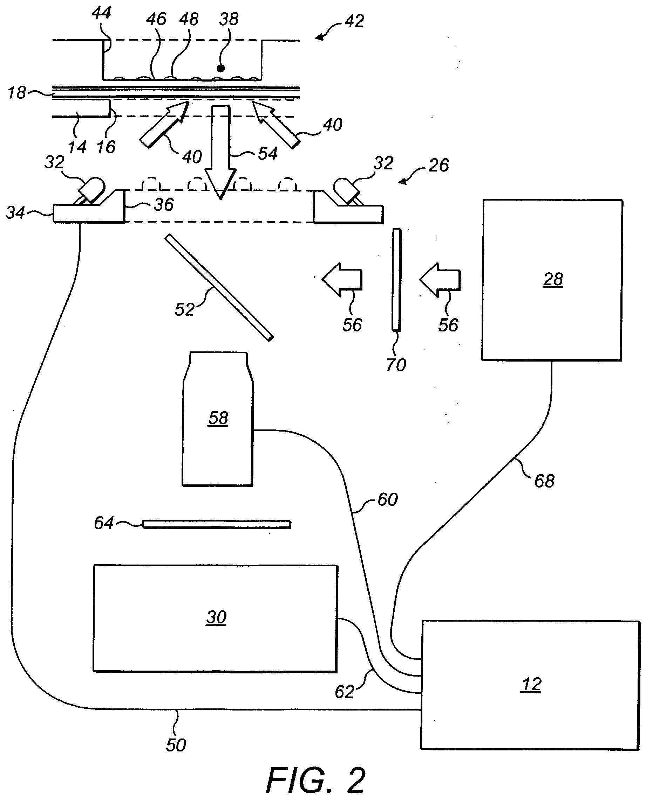

Figure 1 shows a robotic apparatus 10 with its external casing removed to reveal the internal components. The operation of various components of the robotic apparatus 10 is controlled by a computer 12 (which is shown in Figure 2) as described below.

The robotic apparatus 10 has a main platform 14 on which culture plates containing cultured cells are manipulated. As best seen in Figure 2, the main platform 14 is provided with an aperture 16. A clear glass plate 18 lies over the aperture 16. An upper surface of the clear glass plate 18 forms an imaging station 20 where culture plates may be positioned so that cells cultured within the plates can be visualised as described below.

The imaging station 20 also includes positioning guides (not shown) which hold culture plates of standardised configuration in a precise position.

As seen in Figure 1, the robotic apparatus 10 includes an imaging system 22 which is located below the main platform 14 and, more specifically, below the aperture 16. The imaging system 22 is mounted (a described in more detail below) on an optics platform 24 which, in turn, is fixedly mounted relative to the main platform 14.

Turning now to Figure 2, the imaging system 22 includes a first light source 26, a second light source 28 and a camera 30 for obtaining images of cells in culture plates positioned at the imaging station 20. In very general terms, the first light source 26 is used together with the camera 30 to obtain white light images of cells using a dark field configuration. This is useful for determining positions of cell colonies and cells, for determining boundaries of cell colonies and, by taking sequential images, for determining rates of growth of colonies. Again, in general terms, the second light source 28 is used together with the camera 30 for obtaining images of fluorescent or luminescent light emitted from cells. The imaging system 22 and its uses will now be described in more detail.

The first light source 26 comprises a plurality of white light emitting diodes (LEDs) 32. The white light LEDs 32 are mounted on a mounting ring 34 which has a central aperture 36. The optical axes of the LEDs 32 lie on the surface of a common imaginary cone (not shown) such that the respective beams of light emitted by the LEDs 32 converge at a convergence point 38 located at the apex of the imaginary cone. In Figure 2, the inclined arrows 40 show the light paths from two of the LEDs 32 to the convergence point 38.

For the sake of illustration, Figure 2 shows a six well culture plate 42 positioned at the imaging station 20. It will be realised, however, that different types of culture vessel may also be used. One of the wells of the six well culture plate 42 is identified by numeral 44 and lies directly above the imaging system 22. The well 44 has a horizontal culture surface 46 on which are growing a number of adherent cell colonies 48. A liquid growth medium (not shown) is provided in the well 44 and covers the cell colonies 48.

The convergence point 38 of the light beams emitted from the LEDs 32 lies slightly above the culture surface 46 and the cell colonies 48.

The power supply to the LEDs 32 is controlled by the computer 12 as shown schematically by the control line 50. Hence, illumination of the LEDs 32 is controlled by the computer 12.

The imaging system 22 also includes a semi-silvered mirror 52 which is angled at 45 degrees to the horizontal and which is located below the central aperture 36 of the mounting ring 34. In a known manner, a large proportion of light travelling vertically downwards, as shown by the downward arrow 54, passes directly through the semi-silvered mirror 52 without any alteration in direction. On the other hand, a large proportion of light travelling horizontally in the direction shown by the horizontal arrow 56, towards the upper inclined surface of the semi-silvered mirror 52, is reflected vertically upwards by the semi-silvered mirror 52, with the angles of incidence and reflection each being 45 degrees.

The imaging system 22 also includes an autofocus zoom lens 58. One commercially available zoom lens that has been found to be particularly suitable is the Leica Macrofluo Z6APOA zoom lens, although other lens may also be used. The zoom lens 58 receives light passing vertically downwards, in the direction of the downward arrow 54, after passing through the semi-silvered mirror 52. The zoom lens 58 focuses light for reception by the camera 30. The focal length of the zoom lens 58 can be altered and this is controlled by the computer 12, as shown schematically by the control line 60. The optical axis of the zoom lens 58 is aligned with the convergence point 38.

The camera 30 receives light focused by the zoom lens 58. The camera produces an image from the light and passes the image to the computer 12 via the control line 62.

The camera 30 is capable of producing images from relatively low intensity light, such as fluorescent or luminescent light emitted from within cells on culture plates positioned at the imaging station 20. Preferably, the camera 30 is also capable of producing images from such low intensity light sources while covering a relatively large field of view of the culture surface 46 of the culture plate 42 positioned at the imaging station 30. Preferably, an image produced by the camera 30 covers many separate cell colonies 48 (or single cells) adhered to the culture surface 46.

One commercially available camera that has been found to be particularly suitable is the Hamamatsu ORCA Flash 4.0. This camera uses an sCMOS image sensor which provides relatively high sensitivity. In addition, the dimensions of the sensing area of the sCMOS image sensor that is used in the Hamamatsu camera are relatively large (13.312mm x 13.312mm) and this allows the camera 30 to obtain an image covering a relatively large field of view. A further advantage of the Hamamatsu camera is that it has a relatively large number of pixels (2048 x 2048). A relatively large number of pixels enables a high resolution, when necessary. Alternatively, when sensitivity is more important than resolution, the signals from a number of neighbouring pixels may be binned to increase sensitivity.

The following cameras have also been evaluated and found to be suitable: the Apogee ALTA U260; the Andor Neo-sCMOS; and the Andor Neo-sCMOS oem. The dimensions of the sensing areas of the image sensors of these cameras are, respectively: 10.2mm x 10.2mm; 16.6mm x 14mm; and 16.6mm x 14mm. From these investigations, it is concluded that cameras having a sensing area of 100 mm² or greater are preferred.

Cameras having image sensors other than sCMOS image sensors may also be used. For example, cameras having conventional CMOS or CCD image sensors may be used, although the results obtained may not be quite so good.

The imaging system 22 includes a first filter 64. This filter has a bandpass maximum of 516nm. The first filter 64 can be moved out of the light path between the zoom lens 58 and the camera 30 by a suitable motor (not shown) under the control of the computer 12. The first filter 64 serves a purpose described below.

As shown in Figure 2, the second light source 28 is positioned at the same horizontal level as the semi-silvered mirror 52 and directs light in the direction of the horizontal arrows 56 to the upper inclined surface of the semi-silvered mirror 52. Illumination of the second light source 28 is controlled by the computer 12 via the control line 68.

One commercially available light source found to be suitable for the second light source 28 is the Sola 6-LCR SA white light source, although other commercially available light sources may be used.

A second filter 70 which has a bandpass maximum of 495nm is positioned between the second light source 28 and the semi-silvered mirror 52.

The operation of the imaging system 22 will now be described.

As indicated above, the first light source 26 (the LEDs 32) are used in conjunction with the camera 30 to obtain white light images of cells and/or cell colonies 48 growing on the culture surface 46 of a culture plate 42 positioned at the imaging station 20. White light from the LEDs 32 passes up through the aperture 16 in the main platform 14, through the glass plate 18, through the bottom wall of the culture plate 42 towards the convergence point 38. Light scattered downwards by cells colonies 48 and/or single cells passes back down through the culture plate 42, through the glass plate 18, through the aperture 16, through the central aperture 36 to the semi-silvered mirror 52 where the majority of the scattered light passes straight through the semi-silvered mirror 52 to the autofocus zoom lens 58. The zoom lens 58 focuses the light to allow the camera 30 to produce images of the cell colonies 48 and/or single cells on the culture surface 46.

While the first light source 26 and the camera 30 are used to obtain images in this way, the first filter 64 is removed from the light path and plays no part.

Images produced by the camera 30 are passed to the computer 12 for analysis.

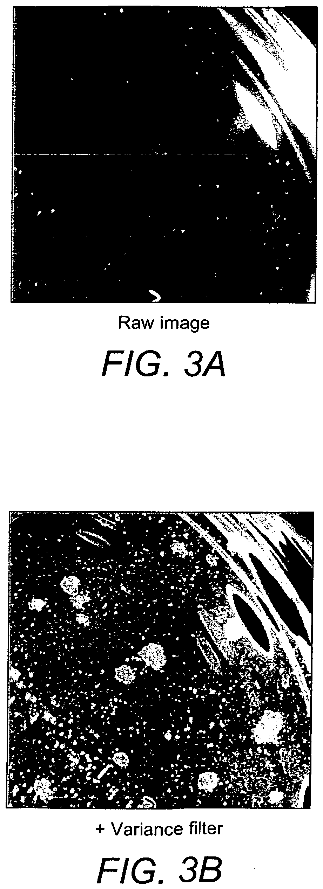

Figures 3A and 3B are white light images of cell colonies 48 obtained in this way. Figure 3A is a raw image and Figure 3B shows the same image after application of a known electronic variance filter to increase the contrast between the cell colonies 48 and the background.

The second light source 28 may be used in combination with the camera 30 to obtain fluorescent images of cell colonies 48 or single cells on the culture plate 42. The first filter 64 and the second filter 70 are chosen with regard to a specific fluorescence system. In this specific example, the first filter 64 and the second filter 70 have been chosen for use with the FLIPR ™ Calcium 5 ™ fluorescent reagent available from Molecular Devices of Sunnyvale, California, USA. This reagent is used to give an indication of cytosolic calcium concentrations. The reagent permeates into cells and binds to cytosolic calcium. The binding of calcium to the reagent increases the fluorescent response of the reagent and so increased cytosolic calcium levels leads to an increase in intracellular fluorescence when the reagent is used. As discussed above, the second filter 70 has a bandpass maximum of 495nn and this is the excitation maximum for the Calcium 5 ™ reagent. The bandpass maximum of 516nm of the first filter 64 corresponds to the fluorescence emission maximum of the Calcium 5 ™ reagent. Clearly, when other fluorescence systems are used, the first and second filters 64, 70 described above may be replaced by other filters having different bandpass maxima suited to the fluorescence system in question. The first and second filters 64, 70 may be carried by filter wheels for changing of filters under the control of the computer 12.

In operation, white light from the second light source 28 is filtered by the second filter 70 so that light with a wavelength maximum of 495nm is reflected upwards by the semi-silvered mirror 52. The reflected light passes through the central aperture 36, the aperture 16, the glass plate 18 and the base of the culture plate 42 to the cell colonies 48 or cells in the culture plate 42. This light excites the Calcium 5 ™ reagent within the cells of the cell colonies 48 (or within single cells). Emitted light from intracellular Calcium 5 ™ reagent passes back down through the base of the culture plate 42, the clear glass plate 18, the aperture 16, the central aperture 36 to the semi-silvered mirror 52. A large proportion of this emitted light passes straight through the semi-silvered mirror 52 into the zoom lens 58 which focuses the light onto the camera 30. In this case, the first filter 64 is positioned in the light path and filters the emitted light as it passes between the zoom lens 58 and the camera 30. Fluorescent images obtained by the camera 30 are passed to the computer 12 for analysis.

While the imaging system 22 is being used to obtain white light images of cell colonies or single cells, using the first light source 26, or while the system is being used to obtain fluorescent images using the second light source 28, the autofocus zoom lens 58 can be operated by the computer 12 so as to zoom in to a particular cell colony 48 or a particular single cell. This may be useful, for example, when it is desired to determine the intracellular location from which fluorescent light is being emitted.

The imaging system 22 is mounted on the optics platform 24 by an optics carriage 72 (see Figure 1) which allows the whole of the imaging system 22 to be moved in any horizontal direction. The optics carriage 72 is connected to the computer 12 so that movement of the imaging system 22 is controlled by the computer 12. Movement of the imaging system 22 in this way allows the imaging system 22 to obtain images of other regions (fields of view) of culture plates 42 located at the imaging station 20.

The position of the optics carriage 72 is related by the processor to an x, y, z coordinate system that is common to a number of robotic components of the robotic apparatus 10.

As indicated above, the culture plate 42 is held at the imaging station 20, by the positioning guides (not shown) at a precise known position. The x, y, z coordinates of this position, using the common x, y, z coordinate system, are stored in the computer 12. Hence, when a cell colony 48 or single cell is imaged by the imaging system 22, the computer 12 can use the position of the colony or cell in the image, the position of the optics carriage 72 when the image was taken, the known geometry of the culture plate 42 and the known location of the culture plate 42 at the imaging station 20 in order to calculate the position of the cell colony or single cell on the culture plate 42. This is useful because it allows the culture plate 42 to be moved to another known location, and so long as the orientation of the culture plate 42 at the new location is known, the precise position of the cell colony or single cell can be determined using the common x, y, z coordinate system. This may be used if, for example, the cells are to be picked from the culture plate 42, as described below, at a different location from the imaging station 20.

Turning back to Figure 1, the robotic apparatus 10 also has a cell picking head 74 located above the main platform 14. The cell picking head 74 is similar to the known cell picking head described in EP1686380 and used in the ClonePix™ 2 robotic apparatus available from Molecular Devices, New Milton, United Kingdom.

As described in EP1686380, the cell picking head 74 is movable over the main platform 14 by a head positioning carriage 76 made up of x, y and z linear positioners that are connected in series and suspended from a gantry 78. The head positioning carriage 76 is controlled by the computer 12 to move the cell picking head over the main platform 14. The processor 12 relates the position of the cell picking head 74 to the common x, y, z coordinate system.

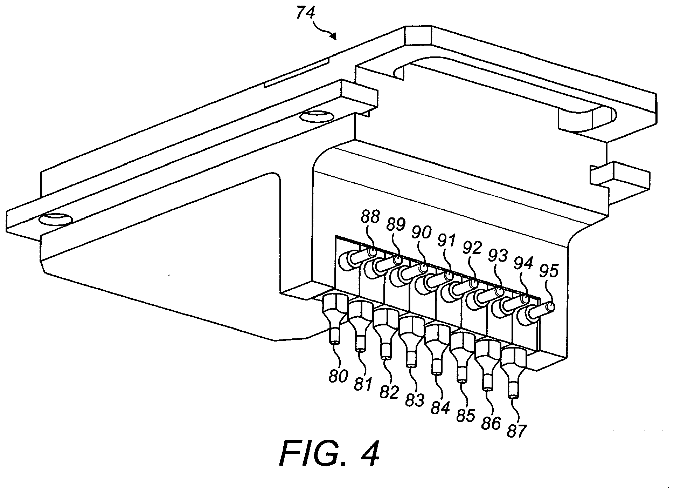

The cell picking head 74 is of conventional design and is shown in Figure 4. As seen in Figure 4, the cell picking head 74 has eight hollow picking needles 80, 81, 82, 83, 84, 85, 86, 87. An interior opening of each hollow picking needle 80, 81, 82, 83, 84, 85, 86, 87 is in fluid communication with a corresponding control port 88, 89, 90, 91, 92, 93, 94, 95. Each control port 88 to 95 is connected via a respective control tube (not shown) to a respective control pump (not shown).

In a conventional manner, each one of the eight picking needles 80 - 87 can be operated, independently, to pick a cell colony or single cell from a culture plate 42 by operating the control pump associated with that picking needle so that the picking needle aspirates the cell colony or single cell. The colony or cell may be adherent or in suspension in a semi-solid medium.

However, each of the eight hollow picking needles 80 - 87 also has a secondary function, which is to dispense a liquid into a culture plate. Each one of the eight hollow picking needles 80 - 87 is connected via its respective control tube and respective control pump to a respective liquid reservoir. Liquid from each of these reservoirs can be pumped to the corresponding picking needle for dispensing. Each picking needle 80 - 87 can dispense a liquid independently of the other picking needles.

Both the operation of the hollow picking needles 80 - 87 to pick cell colonies and single cells, and the operation of the picking needles 80 - 87 to dispense liquids, is controlled by the computer 12.

The robotic apparatus 10 also includes a number of other robotic features which are well known within the art and which are not specifically relevant to the current invention. For example, the robotic apparatus 10 includes means for stacking and storing culture plates and for incubating the stored culture plates at a desired temperature. The robotic apparatus 10 also includes a washing station for cleaning the hollow picking needles 80, 81, 82, 83, 84, 85, 86, 87. Further, the robotic apparatus 10 includes means for replating cells onto new plates and for changing the culture medium on culture plates. Transport means for moving culture plates around the robotic apparatus cells are provided.

The following examples are provided to illustrate how the robotic apparatus 10 described above may be used to select, and optionally pick, a cultured clonal cell colony or a cultured single cell having a desired characteristic. The following Examples 1 to 5 are not in accordance with the invention but provide additional relevant information on the operation of the robotic apparatus.

### Example 1

The following examples make use of the fluorescent Calcium 5 ™ reagent to allow cytosolic calcium levels to be assessed using fluorescent imaging. The purpose of this example is simply to demonstrate that the Calcium 5 ™ reagent has no appreciable toxicity to the cells used in the following examples.

This example makes use of cultured Chinese Hamster Ovary KI (CHO-K1) cells which express the Angiotensin II Type 1 (AT1) receptor. The AT1 receptor is a G-protein coupled receptor (GPCR). It binds to the ligand Angiotensin II. The activated AT1 receptor binds to G_{q/11} which in turn activates Phospholipase C, which in turn increases cytosolic calcium concentrations.

A suspension of AT1 expressing CHO-K1 cells in liquid culture medium was plated into the six wells of a standard six well culture plate. The plate was incubated until the cells had formed small discrete colonies. Next, a solution of Calcium 5 ™ reagent, in the same liquid culture medium, was added to three of the six wells. A corresponding volume of the liquid culture medium was added to the other three wells. In those wells to which it was added, the concentration of the Calcium 5 ™ reagent was as recommended by the manufacturer. The cells were then incubated with the reagent for one hour at 37°C. After the incubation, the cells were incubated for another hour, this time at room temperature. This second incubation is to simulate the environmental conditions present when cells are imaged and picked in the robotic apparatus 10. After the second incubation, the liquid culture medium was removed from all six wells and was replaced with fresh liquid culture medium. Finally, the plate was returned to 37°C and the cells were allowed to grow for 7 days.

During the 7 day incubation, growth of the cells in the six wells was monitored periodically using a CloneSelect ™ Imager (available from Molecular Devices (New Milton) Ltd). Cell growth could equally have been monitored using the imaging system 22 of the robotic apparatus 10 described above. In this latter case, the first light source 26 and the camera 30 would be used to produce white light images of the cells and these images would be analysed by the computer 12 to determine the percentage coverage of the culture surface 46 by the cells.

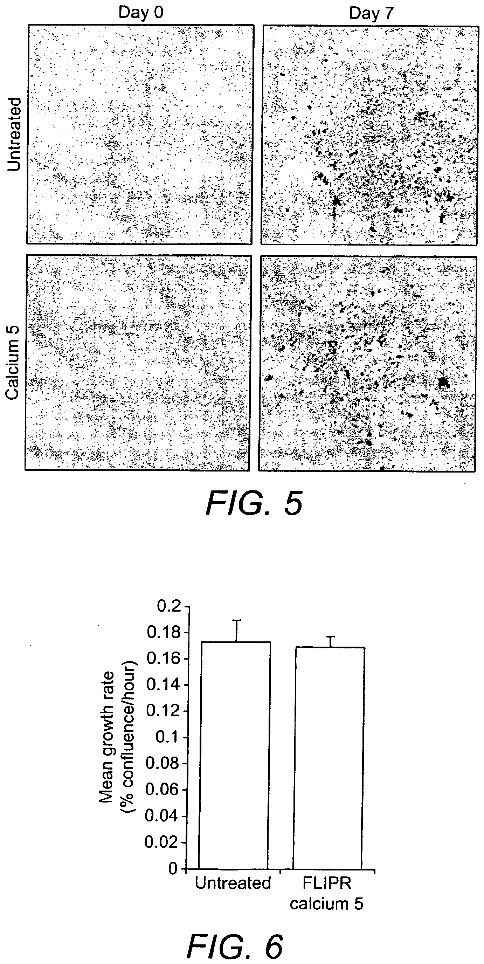

Images taken at Day 0 and Day 7 of the incubation are shown in Figure 5. In Figure 5, the upper two images represent the coverage of the cells, at Day 0 and Day 7 respectively, in a well that was not treated with the Calcium 5 ™ reagent. The lower two images represent the coverage of the cells, at Day 0 and Day 7 respectively, in a well that was treated with the Calcium 5 ™ reagent. As shown, similar cell growth took place both in the wells that had not been treated and also in the wells that had been treated.

Figure 6 shows the results of the experiment graphically, with mean growth rate being shown on the y-axis for both untreated cells and also for the cells that had been treated with the Calcium 5 ™ reagent. No significant difference was observed.

This indicates that there is no significant inhibition of growth caused by treating the cells with the Calcium 5 ™ reagent.

### Example 2

This example demonstrates that the imaging system 22 of the robotic apparatus 10 is sufficiently sensitive to detect fluorescent light emitted from within cells by the Calcium 5 ™ reagent so as to allow detection of an increase in cytosolic calcium concentration.

The example makes use of CHO-K1 cells that express the AT1 receptor as well as CHO-K1 cells that do not express this receptor.

AT1 expressing CHO-K1 cells and non-expressing CHO-K1 cells were plated and grown in separate culture plates using an appropriate, commercially available liquid culture medium. The culture plates were incubated until the cells were observed to have formed small discrete adherent colonies and the following steps were performed on each culture plate so that the AT1 expressing and non-expressing cells were treated identically.

Firstly, the cell colonies were loaded with Calcium 5 ™ reagent by incubating the appropriate culture plate with the reagent at 37°C for one hour as described above in Example 1. Calcium 5 ™ reagent enters into the cultured cells.

The following steps were performed with the culture plate being treated positioned at the imaging station 20.

After the loading with Calcium 5 ™ reagent, cell colonies were imaged using the first light source 26 and the camera 30 to produce white light images that were passed to the computer 12 for analysis. During analysis, the computer 12 determined the positions of the imaged colonies in the culture plate as well as the boundaries of the colonies.

After the white light imaging, the cell colonies on the culture plate were imaged using the second light source 28 and the camera 30. These images were passed to the computer 12 and stored. The images taken at this stage show the baseline levels of fluorescence emitted by the Calcium 5 ™ reagent located intracellularly within cell colonies.

The computer 12 then operated the head positioning carriage 76 to position the fifth hollow picking needle 84 over the culture plate (which had previously been de-lidded). The hollow picking needle 84 was connected via the corresponding control tube and the corresponding control pump to a reservoir containing a solution of Angiotensin II in culture medium. The computer 12 then operated the control pump to cause the hollow picking needle 84 to dispense Angiotensin II solution into the culture plate. The volume of solution dispensed was approximately one quarter of the volume of culture medium in the culture plate prior to dispensing. The dispensing of a relatively large volume of solution (such as ¼ of the volume of the culture medium in the plate) allows the Angiotensin II to reach all of the cell colonies in the culture plate at approximately the same time. The fact that concentration of Angiotensin II may vary in different areas of the plate is acceptable.

Immediately after the dispensing of the Angiotensin II solution, the imaging system 22 was operated to obtain sequential fluorescent images of the culture plate showing fluorescent light emitted from intracellular Calcium 5 ™ reagent in the cell colonies 48 being imaged. This involved illumination of the colonies using the second light source 28 and the second filter 70 and detection of emitted light using the camera 30 and the first filter 64, the imaging system 22 being operated as described above.

The final average concentration of Angiotensin II in the culture plate was 10.5mM.

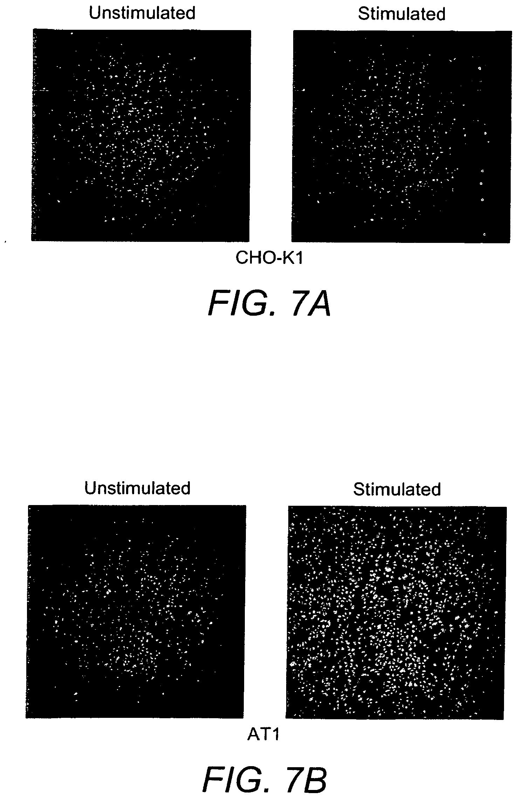

Details from fluorescent images obtained during this experiment are shown in Figures 7A and 7B. The two images of Figure 7A show results for CHO-K1 cells that do not express the AT1 receptor. Each of the two images of Figure 7A cover the same cell colony. The two images of Figure 7B show results for the AT1 expressing CHO-K1 cells. Looking first at Figure 7B, the left hand image is a baseline fluorescent image taken before addition of Angiotensin II and covering one cell colony. There is a relatively small amount of intracellular fluorescence evident in this image. The right hand image of Figure 7B is a fluorescent image covering the same clonal cell colony covered by the left hand image and taken at approximately 16 seconds after the addition of Angiotensin II. The right hand image shows numerous points of light. Each point of light represents fluorescent light being emitted by the Calcium 5 ™ reagent from within a separate cell within the cell colony. The level of fluorescence is significantly greater than in the left hand baseline image. Hence, the Angiotensin II triggered an increase in cytosolic calcium concentration in AT1 expressing CHO-K1 cells and this could be readily detected using the Calcium 5 ™ reagent and the imaging system 22 of the robotic apparatus 10.

Looking now at Figure 7A, the left hand image is the baseline image prior to addition of Angiotensin II and the right hand image is an image taken approximately 16 seconds after addition of Angiotensin II. The images are virtually identical, as is expected. In the absence of AT1 receptor, the addition of Angiotensin II does not cause an increase in cytosolic calcium concentration.

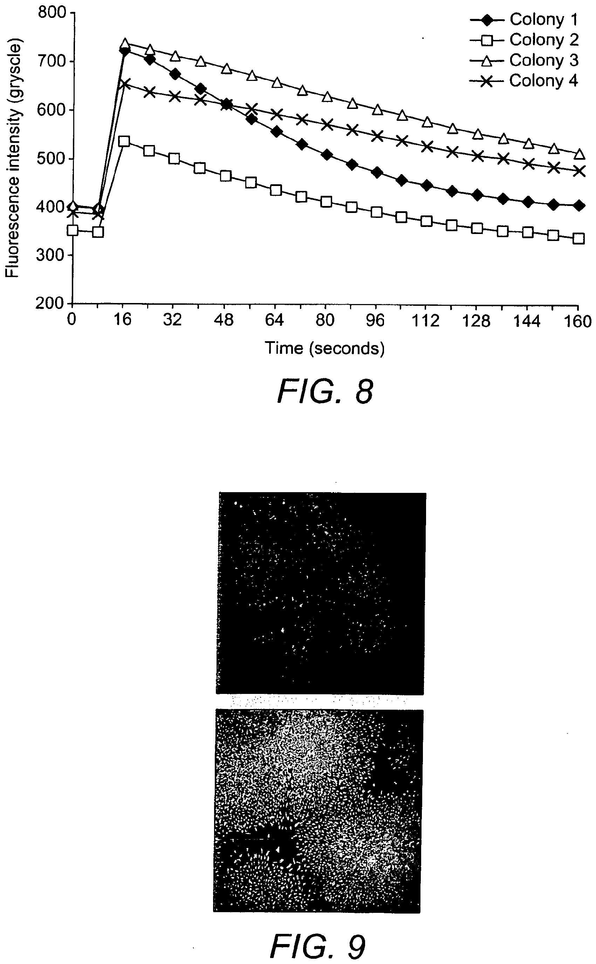

Figure 8 shows fluorescence intensity levels determined by the computer 12, from analysing images obtained by the imaging system 22, for four randomly selected cell colonies of the AT1 expressing cells. In Figure 8, each point on the graph represents a fluorescence intensity level determined for an individual cell colony from a single fluorescence image obtained by the imaging system 22. The fluorescence levels at t=0 are the fluorescence levels in the baseline image. After addition of Angiotensin II, images were taken every 8 seconds and the graph shows the relative fluorescence intensity, for each of the four colonies, obtained for each of the sequential images. As will be seen from Figure 8, intracellular fluorescence peaked at about 16 seconds after addition of Angiotensin II and then decreased relatively steadily through the following 140 seconds.

### Example 3

In Example 2, by comparing the fluorescence images with the white light images, it was evident that all or substantially all of the AT1 expressing cell colonies showed an increase in intracellular fluorescence after addition of Angiotensin II. This is to be expected as the AT1 expressing CHO-K1 cells used in Example 2 are from a stable AT1 expressing cell line.

It will be appreciated, however, that the same methodology may be applied to a heterogeneous mix of cell colonies (or single cells), and that the methodology may be used to select a cell colony (or single cell) having a desired characteristic from the mix.

The following is an example of how this can be done.

A cell line that does not express the AT1 receptor is transfected with a genetic construct designed to give AT1 expression. As is well known, the transfection process is inefficient. After transfection, many cells will not express AT1 at all, and for those cells which do express AT1, there will be a large variation in the degree of expression.

A suspension of the transfected cells is plated and incubated so as to form discrete cell colonies. Before plating, the cell suspension is diluted sufficiently to ensure that most if not all of the colonies that grow on the plate are derived from a single cell and so are clonal. The culture plate containing the clonal cell colonies is subjected to the same methodology used in Example 2.

During this process, the relative fluorescence intensity obtained for each clonal colony after addition of Angiotensin II solution is compared to the relative fluorescence intensity obtained for the same colony prior to addition (i.e. the baseline level). An increase in intracellular fluorescence indicates that the cytosolic calcium concentration has increased and this, in turn, is a marker for the expression of functional AT1 receptor.

Those clonal cell colonies which demonstrate an increase in intracellular fluorescence after addition of Angiotensin II are selected by the computer 12 and the computer 12 determines the position of those colonies on the culture plate using white light imaging.

Knowing the positions of the cell colonies that exhibited an increase in intracellular fluorescence, those colonies can be picked using the cell picking head 74 under the control of the computer 12 and the cells of each picked colony can be sub-cultured and subjected to further analysis to determine whether the clone exhibits the desired expression of AT1 receptor.

The colonies that do not demonstrate an increase in intracellular fluorescence are not picked and not subjected to further examination. This saves a great deal of time and resources that might otherwise be expended in analysing unsuitable clones.

### Example 4

This example makes use of cells derived from a single colony of AT1 expressing CHO-K1 cells picked by the cell picking head 74 after the procedure of Example 2. The cells from the picked colony were suspended and plated in a fresh plate with culture medium. The process of Example 2 was then repeated on the freshly plated cells. Images taken during Example 4 are shown in Figure 9. In Figure 9, the top image is a fluorescent baseline image obtained before addition of Angiotensin II. The bottom image is a fluorescent image obtained approximately 20 seconds after addition of Angiotensin II. As will be seen, the levels of fluorescence in the bottom image are significantly greater than those in the top image.

This example demonstrates that it is possible to expose cells to Calcium 5 ™ reagent, stimulate them with Angiotensin II, and pick them, and that the cells are still responsive to Angiotensin II and still demonstrate an increase in intracellular fluorescence after re-plating.

### Example 5

With any imaging system of the type described above, there is a trade-off between sensitivity to fluorescence originating from individual colonies or cells, and field of view. The field of view may be increased with a decrease in sensitivity to individual colonies or cells (eg by zooming out) and, conversely, sensitivity may be increased with a decrease in field of view (eg by zooming in). If, in order to obtain the required sensitivity, the field of view does not cover the complete culture surface of a culture plate, the optics carriage 72 may move the imaging system 22 so that the complete culture surface 46 can be imaged by taking a series of images taken at different positions of the optics carriage 72.

In experiments performed with the robotic apparatus 10 described above, a single well 44 of a standard six well culture plate was imaged using nine separate overlapping images arranged in a 3 x 3 matrix. It was found that the nine overlapping images could be generated within a total time period of 13 seconds; including a 200ms exposure time for each image, and the time taken to download the images to the computer 12 and to move the optics carriage 72 to the required positions.

## Claims

1. A method of selecting a cultured cell colony or a cultured single cell having functional copies of a transcription factor, comprising:
providing a plurality of cultured cell colonies or of cultured single cells in a common culture space wherein the plurality is heterogeneous in respect of presence of functional copies of the transcription factor;
adding a ligand for the transcription factor to the cell colonies or to the single cells, while the cell colonies or the single cells are in the common culture space, wherein the addition of the ligand elicits a change in activity of the transcription factor occurring on migration of the transcription factor from the cytoplasm to the nucleus in at least one of the cell colonies or the single cells, the change in the activity being indicative of possession of functional copies of the transcription factor by the or each cell colony or single cell in which the change in the activity is elicited;
assessing individual ones of the cell colonies or of the single cells, in the common culture space, for a detectable change in location of the transcription factor;
selecting at least one but not all of the cell colonies or of the single cells, wherein said selection takes account of said assessment to determine expected possession of functional copies of the transcription factor, wherein the or each selected cell colony or single cell is expected to have functional copies of the transcription factor on the basis of said assessment; and
providing an indication of the spatial position of at least one of the cell colonies or of the single cells that are selected.

2. A method according to claim 1, wherein the assessment comprises detecting fluorescence or luminescence.

3. A method according to any preceding claim, further including picking the or one of the selected cell colonies or single cells from the common culture space and culturing the picked cell colony or single cell.

4. A computer program comprising instructions for directing a robotic apparatus to perform a method according to any preceding claim.

5. A computer readable medium having stored thereon a computer program according to claim 4.

6. A robotic apparatus including a processor and a storage medium, the storage medium having stored thereon a computer program according to claim 4. the processor being connected to the storage medium for running of the computer program on the processor.

## Patentansprüche

1. Verfahren zum Auswählen einer kultivierten Zellkolonie oder einer kultivierten Einzelzelle, die funktionelle Kopien eines Transkriptionsfaktors aufweist, umfassend:
Bereitstellen einer Vielzahl kultivierter Zellkolonien oder kultivierter Einzelzellen in einem gemeinsamen Kulturraum, wobei die Vielzahl in Bezug auf die Anwesenheit von funktionellen Kopien des Transkriptionsfaktors heterogen ist;
Zusetzen eines Liganden für den Transkriptionsfaktor zu den Zellkolonien oder zu den Einzelzellen, während sich die Zellkolonien oder die Einzelzellen in dem gemeinsamen Kulturraum befinden, wobei der Zusatz des Liganden eine Veränderung der Aktivität des Transkriptionsfaktors hervorruft, die bei der Migration des Transkriptionsfaktors vom Zytoplasma zum Kern in mindestens einer von den Zellkolonien oder den Einzelzellen auftritt, wobei die Veränderung der Aktivität auf den Besitz von funktionellen Kopien des Transkriptionsfaktors durch die oder jede Zellkolonie oder Einzelzelle hindeutet, in der die Veränderung der Aktivität hervorgerufen wird;
Beurteilen einzelner der Zellkolonien oder der Einzelzellen in dem gemeinsamen Kulturraum für eine nachweisbare Ortsveränderung des Transkriptionsfaktors;
Auswählen mindestens einer, aber nicht aller Zellkolonien oder aller Einzelzellen, wobei die Auswahl die Beurteilung berücksichtigt, um den erwarteten Besitz von funktionellen Kopien des Transkriptionsfaktors zu bestimmen, wobei von der oder jeder ausgewählten Zellkolonie oder Einzelzelle erwartet wird, dass sie funktionelle Kopien des Transkriptionsfaktors auf Grundlage dieser Beurteilung aufweist; und
Bereitstellen eines Hinweises bezüglich der räumlichen Position von mindestens einer der Zellkolonien oder der Einzelzellen, die ausgewählt werden.

2. Verfahren nach Anspruch 1, wobei die Beurteilung Erfassen von Fluoreszenz oder Lumineszenz umfasst.

3. Verfahren nach einem der vorhergehenden Ansprüche, ferner beinhaltend Herausnehmen der oder einer der ausgewählten Zellkolonien oder Einzelzellen aus dem gemeinsamen Kulturraum und Kultivieren der herausgenommenen Zellkolonie oder Einzelzelle.

4. Computerprogramm, umfassend Anweisungen zum Anleiten einer Robotervorrichtung, ein Verfahren nach einem der vorhergehenden Ansprüche durchzuführen.

5. Computerlesbares Medium, auf dem ein Computerprogramm nach Anspruch 4 gespeichert ist.

6. Robotervorrichtung, die einen Prozessor und ein Speichermedium beinhaltet, wobei auf dem Speichermedium ein Computerprogramm nach Anspruch 4 gespeichert ist und der Prozessor mit dem Speichermedium zum Ausführen des Computerprogramms auf dem Prozessor verbunden ist.

## Revendications

1. Procédé de sélection d'une colonie de cellules de culture ou d'une cellule unique de culture ayant des copies fonctionnelles d'un facteur de transcription, comprenant :
la fourniture d'une pluralité de colonies de cellules de culture ou de cellules uniques de culture dans un espace de culture commun dans lequel la pluralité est hétérogène en ce qui concerne la présence de copies fonctionnelles du facteur de transcription ;
l'ajout d'un ligand pour le facteur de transcription aux colonies de cellules ou aux cellules uniques, tandis que les colonies de cellules ou les cellules uniques se trouvent dans l'espace de culture commun, dans lequel l'ajout du ligand provoque un changement de l'activité du facteur de transcription se produisant lors de la migration du facteur de transcription du cytoplasme au noyau dans au moins l'une des colonies de cellules ou des cellules uniques, le changement de l'activité indiquant la possession de copies fonctionnelles du facteur de transcription par la ou chaque colonie de cellules ou cellule unique dans laquelle le changement de l'activité est provoqué ;
l'évaluation de certaines des colonies de cellules ou des cellules uniques, dans l'espace de culture commun, pour un changement détectable de l'emplacement du facteur de transcription ;
la sélection d'au moins l'une mais pas de la totalité des colonies de cellules ou des cellules uniques, dans lequel ladite sélection tient compte de ladite évaluation pour déterminer la possession attendue de copies fonctionnelles du facteur de transcription, dans lequel on s'attend à ce que la ou chaque colonie de cellules ou cellule unique sélectionnée ait des copies fonctionnelles du facteur de transcription sur la base de ladite évaluation ; et
la fourniture d'une indication de la position spatiale d'au moins l'une des colonies de cellules ou des cellules uniques qui sont sélectionnées.

2. Procédé selon la revendication 1, dans lequel l'évaluation comprend la détection de la fluorescence ou de la luminescence.

3. Procédé selon une quelconque revendication précédente, comprenant en outre le prélèvement de la ou de l'une des colonies de cellules ou des cellules uniques sélectionnées à partir de l'espace de culture commun et la culture de la colonie de cellules ou de la cellule unique prélevée.

4. Programme informatique comprenant des instructions pour diriger un appareil robotisé pour mettre en oeuvre un procédé selon une quelconque revendication précédente.

5. Support lisible par ordinateur sur lequel est stocké un programme informatique selon la revendication 4.

6. Appareil robotisé comprenant un processeur et un support de stockage, le support de stockage sur lequel est stocké un programme informatique selon la revendication 4, le processeur étant connecté au support de stockage pour l'exécution du programme informatique sur le processeur.
